# FASCICULE DE BREVET EUROPEEN

(11) **EP 2 683 334 B1**
(45) Date de publication et mention de la délivrance du brevet: **24.06.2015**
(21) Numéro de dépôt: 12710351.3
(22) Date de dépôt: 05.03.2012
(51) Int. Cl.: A61F 2/30, A61B 17/86, A61F 2/46, A61F 2/02

(54) **DISPOSITIF DE SURVEILLANCE D'UNE PROTHESE MEDICALE ET DU CORPS HUMAIN**
VORRICHTUNG ZUR ÜBERWACHUNG EINES MEDIZINISCHE PROTHESE UND DES MENSCHLICHEN KÖRPERS
DEVICE FOR MONITORING A MEDICAL PROSTHESIS AND THE HUMAN BODY

(30) Priorité: 07.03.2011 FR 1151857
(43) Date de publication de la demande: 15.01.2014
(73) Titulaire: Lape Medical, 74950 Scionzier (FR); Reucirs, 69110 Sainte Foy Les Lyon (FR)
(72) Inventeur: GRADEL, Thomas, F-74970 Marignier (FR); WEIL, Gérard, F-69110 Sainte Foy Les Lyon (FR); PIFFAULT, Lionel, F-17800 Montils (FR); GAUCHER, Fabien, F-69001 Lyon (FR)
(74) Mandataire: Poncet, Jean-François
(86) Numéro de dépôt international: PCT/IB2012/051030
(87) Numéro de publication internationale: WO 2012/120439

(56) Documents cités:
- WO-A2-2006/055547
- DE-A1-102008 005 180
- US-A1- 2007 179 568
- US-A1- 2007 179 739
- US-A1- 2010 100 011

## Description

### DOMAINE TECHNIQUE DE L'INVENTION

La présente invention concerne le domaine des prothèses, et concerne plus particulièrement le domaine des prothèses dites « intelligentes ».

Afin de surveiller le comportement d'une prothèse implantée dans le corps d'un patient, par exemple son ostéo-intégration ou son usure, il a été tenté d'inclure des moyens de capteur dans la prothèse, avec des moyens de transmission sans fil permettant de transmettre des données à l'extérieur du corps du patient.

On connaît notamment à cet effet le document WO 2009/095768.

L'intégration de moyens de capteur dans une prothèse oblige cependant à une re-conception totale des prothèses classiques qui donnaient jusque là satisfaction. Il est entre autres nécessaire de s'assurer que l'intégration des moyens de capteur ne nuit pas à la résistance mécanique de la prothèse. Cela nécessite de longues et onéreuses études de requalification de la prothèse auprès des organismes compétents.

Il n'est en outre pas toujours possible d'intégrer des moyens de capteur dans les prothèses existantes, du fait de leur forme ou de leur section.

Un autre inconvénient est que, en cas de défaillance des moyens de capteur, il est nécessaire de retirer la prothèse implantée alors même que celle-ci peut parfaitement fonctionner. Le retrait d'une prothèse occasionne souvent des détériorations supplémentaires, ce qui conduit généralement à implanter par la suite une prothèse dite « de reprise » qui est le plus souvent de taille plus encombrante et moins performante.

Un autre inconvénient est que le patient est obligé de décider avant la pose de la prothèse s'il accepte ou non, de façon définitive, une surveillance par des moyens de capteur.

Un autre inconvénient est que, si le patient décide ou est contraint après la pose de la prothèse d'accepter une surveillance par des moyens de capteur, il est alors nécessaire de procéder au retrait de la prothèse implantée pour la remplacer par une prothèse à moyens de capteur. Cette prothèse à moyens de capteur est alors de type prothèse de reprise.

Un autre inconvénient est que, si une pathologie imprévue apparaît, il est possible que les moyens de capteur présents dans la prothèse implantée soient inadaptés pour surveiller celle-ci. Il est donc nécessaire de pouvoir choisir et adapter les moyens de capteur à utiliser en fonction d'une pathologie prévisible ou déclarée, et ce même après que la prothèse ait été implantée.

A cela s'ajoute un inconvénient de coût, une prothèse équipée de moyens de capteur étant très onéreuse.

Un autre inconvénient est que les moyens de capteur sont généralement alimentés par une batterie qui a une durée de vie limitée et qu'il faut pouvoir changer régulièrement. Et des maintenances et/ou remplacements des moyens de capteur peuvent nécessiter leur retrait complet et réimplantation. La masse osseuse à laquelle est fixée la prothèse et/ou les moyens de capteurs est ainsi dégradée ou fragilisée à plusieurs reprises. Tel est notamment le cas dans les documents US 2007/0179739, US 2007/0179568, US 2010/0100011 et WO 2006/055547.

### EXPOSE DE L'INVENTION

Un premier but de l'invention est de permettre une surveillance d'une prothèse par des moyens de capteur quelle que soit la forme ou la configuration de la prothèse.

Un autre but de l'invention est de permettre au patient de faire un choix réversible de surveillance ou non de la prothèse, sans conséquence sur l'opération de pose de la prothèse à effectuer ou effectuée.

Un autre but de l'invention est de permettre des maintenances et/ou remplacements en limitant la dégradation et la fragilisation de la masse osseuse environnante.

Pour atteindre ces buts, ainsi que d'autres, l'invention propose un dispositif de surveillance d'une prothèse médicale et du corps humain, comprenant :
- des moyens de capteur, aptes à générer des signaux en fonction de sollicitations environnantes,
- des moyens de traitement, aptes à traiter les signaux émis par les moyens de capteur pour les convertir en données,
- des moyens de mémorisation, aptes à mémoriser les données,
- des moyens de transmission sans fil des données mémorisées,
- des moyens d'alimentation en énergie électrique,
dans lequel lesdits moyens de capteur, de traitement, de mémorisation, de transmission et d'alimentation sont inclus dans au moins un boîtier en matériau biocompatible,
et dans lequel ledit au moins un boîtier est solidarisé de façon réversible à des moyens de fixation par des moyens de verrouillage, lesdits moyens de fixation étant destinés et adaptés pour être fixés à un os.

Un tel dispositif permet de résoudre les inconvénients susmentionnés.

En particulier, les moyens de capteur sont parfaitement indépendants de la prothèse à surveiller, tout en pouvant être placés à proximité immédiate de celle-ci pour la surveiller efficacement. Les prothèses connues et reconnues pour leur bon fonctionnement peuvent être utilisées normalement, sans avoir à subir de modifications ou de requalifications.

L'installation des moyens de capteur peut être effectuée pendant ou après implantation de la prothèse.

Une intervention sur les moyens de capteur n'a aucun impact sur la prothèse implantée.

Le retrait du boîtier pour son remplacement ou sa maintenance est effectué de façon simple grâce aux moyens de verrouillage réversibles. Les moyens de fixation restent à demeure dans la masse osseuse et peuvent ainsi s'ostéo-intégrer de façon satisfaisante.

Avantageusement, le dispositif de surveillance comporte au moins un premier boîtier, contenant les moyens d'alimentation en énergie électrique, et qui est relié à un deuxième boîtier par des moyens de conduction électrique.

La source d'énergie électrique, qui est en général l'élément le plus encombrant lorsque l'on souhaite disposer d'une durabilité importante dans le temps, peut être placée à distance de la prothèse, à un endroit et dans une disposition qui ne gênent pas. En revanche, les moyens de capteur pourront être contenus dans le deuxième boîtier qui pourra alors présenter un faible encombrement pour être placé dans le voisinage direct de la prothèse afin de mieux surveiller celle-ci.

De préférence, on peut prévoir que :
- le dispositif comporte au moins un premier boîtier et un deuxième boîtier,
- au moins l'un des deux boîtiers comporte des moyens de capteur adaptés pour détecter la position relative de l'autre boîtier.

En fixant les boîtiers à deux os situés de part et d'autre d'une articulation prothétique, on peut ainsi relever les mouvements de l'articulation en nombre de cycles et/ou en amplitude, comme il est par exemple expliqué dans le document WO 2010/003824. On dispose alors de données pour apprécier la sollicitation de la prothèse, la qualité de fonctionnement de la prothèse et/ou sa durée de fonctionnement restante.

Avantageusement, on peut prévoir que :
- le dispositif comporte au moins un premier et un deuxième boîtiers,
- le dispositif comporte des moyens de communication entre les premier et deuxième boîtiers, permettant à l'un et/ou l'autre des boîtiers de recevoir des données de l'autre boîtier,
- au moins l'un des boîtiers comporte des moyens de transmission sans fil des données stockées pour émettre les données reçues de l'autre boîtier.

De préférence, les moyens de capteur peuvent être sensibles à des sollicitations électriques, chimiques, mécaniques ou optiques.

Parmi les capteurs mécaniques, on pourra utiliser, selon les données que l'on cherche à obtenir : un gyromètre, un inclinomètre (de préférence de type magnétomètre), ou encore un accéléromètre.

On pourra également prévoir un capteur de pression, adapté pour apprécier la pression dans le milieu environnant la prothèse.

Avantageusement, les moyens de capteur peuvent comporter un capteur de mesure de la conductivité du milieu environnant. Un tel capteur pourra notamment permettre de détecter une usure prématurée d'une prothèse métallique, cette usure provoquant le largage et la dispersion de particules métalliques dans le corps humain au voisinage de la prothèse.

Un capteur de température peut être utilisé afin de repérer la présence d'une éventuelle infection, inflammation ou autre réaction à manifestation thermique du corps au voisinage de la prothèse.

De préférence, les moyens de capteur peuvent comporter un capteur de mesure du pH.

Avantageusement, les moyens de capteur peuvent comporter un capteur optique adapté pour détecter la couleur du milieu environnant. La détection d'un changement de couleur peut manifester une réaction physico-chimique signe d'un dysfonctionnement, d'une infection ou d'une pathologie.

De préférence, les moyens d'alimentation en énergie électrique peuvent comprendre :
- des moyens de conversion des accélérations subies par ledit au moins un boîtier pour les convertir en énergie électrique,
- des moyens de stockage de l'énergie électrique convertie.

On dispose ainsi d'une source d'énergie renouvelable et qui est disponible lorsqu'il est important d'effectuer des mesures et/ou détections de mouvements.

Avantageusement, on peut prévoir que :
- le dispositif comporte des moyens d'inhibition de certains moyens de capteur et/ou moyens de traitement,
- les moyens d'inhibition sont conformés pour inhiber le fonctionnement de ces moyens de capteur et/ou moyens de traitement pendant des plages horaires prédéterminées.

Les moyens d'inhibition permettent d'économiser de l'énergie, et d'augmenter ainsi la durée de vie du dispositif dans le cas d'une source d'énergie non renouvelable du type batterie par exemple.

De préférence, on peut prévoir que :
- le dispositif comporte des moyens d'inhibition de certains moyens de capteur et/ou moyens de traitement,
- les moyens d'inhibition sont conformés pour n'autoriser le fonctionnement des moyens de traitement qu'après que des moyens de capteur non inhibés aient détecté des mouvements pendant une durée supérieure à une durée prédéterminée.

On ne détectera ainsi que les mouvements d'amplitude supérieure à une amplitude prédéterminée, sans tenir compte des mouvements se produisant pendant des séquences trop courtes pour avoir un impact sur la durée de vie et le fonctionnement de la prothèse.

Toujours dans le but de ne pas utiliser inutilement de l'énergie pour la surveillance et l'analyse de mouvements peu importants ou à influence peu significative, on peut prévoir que :
- le dispositif comporte des moyens d'inhibition de certains moyens de capteur et/ou moyens de traitement,
- les moyens d'inhibition sont conformés pour n'autoriser le fonctionnement des moyens de traitement qu'après que des moyens de capteur non inhibés aient détecté des mouvements d'amplitude supérieure à une amplitude prédéterminée.

Avantageusement, on peut prévoir que :
- les moyens de mémorisation des données contiennent des données de référence prédéterminées,
- le dispositif comporte des moyens de comparaison, adaptés pour comparer aux données de référence prédéterminées les données issues du traitement des signaux émis par les moyens de capteur,
- en cas de non-conformité des données issues du traitement des signaux émis par les moyens de capteur avec les données de référence prédéterminées, un signal d'anomalie est généré et stocké dans les moyens de mémorisation.

En scrutant les signaux d'anomalie stockés dans les moyens de mémorisation, on peut ainsi surveiller en continu la conformité du fonctionnement de la prothèse ou de son milieu environnant avec des données de référence prédéterminées correspondant à un fonctionnement normal ou à un milieu environnant normal. Tout écart par rapport à une situation normale est ainsi détecté.

Pour assurer une liaison la plus intime possible avec l'os sur lequel est fixé le dispositif, ledit au moins un boîtier peut comporter une face destinée à venir en appui contre l'os au moyen d'une conformation adaptée à la forme extérieure de l'os.

En alternative, afin de disposer d'un boîtier de forme unique adaptable à tout os, on peut prévoir que le dispositif comporte une cale comprenant :
- une première face destinée à venir en appui contre l'os au moyen d'une conformation adaptée à la forme extérieure de l'os,
- une deuxième face, sensiblement opposée à la première face, destinée à venir en appui contre une face dudit au moins un boîtier au moyen d'une conformation adaptée à la face dudit au moins un boîtier.

Avantageusement, les moyens de fixation dudit au moins un boîtier peuvent comprendre :
- une unique vis de solidarisation dudit au moins un boîtier à l'os,
- des moyens de blocage en rotation dudit au moins un boîtier par rapport à la vis de solidarisation ou par rapport à l'os.

Une unique vis de solidarisation permet d'affecter l'os le moins possible tandis que les moyens de blocage en rotation assurent une orientation constante du boîtier, notamment pour que les détection et analyse de mouvements se fassent selon des directions constantes.

De préférence, on peut prévoir que :
- les moyens de fixation comportent au moins une vis de solidarisation ou, le cas échéant, une unique vis de solidarisation,
- la vis de solidarisation comprend une partie inférieure à filet à moyens d'ostéo-intégration,
- la vis de solidarisation comprend une partie supérieure adaptée pour coopérer avec les moyens de verrouillage dudit au moins un boîtier sur la partie supérieure afin de retenir ledit au moins un boîtier.

La vis de solidarisation est ainsi fixée de manière définitive dans l'os pour une tenue parfaite du boîtier qui pourra malgré tout être démonté et remonté en cas de besoin de maintenance ou de remplacement, sans affecter la masse osseuse environnante.

De préférence, on peut prévoir que :
- ledit au moins un boîtier est un boîtier tubulaire creux, s'étendant selon une direction longitudinale, apte à être verrouillé dans un manchon sensiblement tubulaire à paroi extérieure munie d'un filetage extérieur,
- le boîtier tubulaire et/ou le manchon sensiblement tubulaire comporte une extrémité munie de moyens d'entraînement en rotation autour de la direction longitudinale.

Le boîtier se présente ainsi sensiblement sous la forme d'une vis directement implantable par vissage dans l'os du patient. Une fois implanté, le boîtier peut présenter un faible dépassement par rapport à l'os, pour limiter le risque de conflit avec les tissus et chairs environnants.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue de dessus d'un dispositif de surveillance d'une prothèse médicale et du corps humain, selon un premier mode de réalisation de l'invention ;
- les figures 2 et 3 sont des vues schématiques de face en coupe de deux variantes du dispositif de la figure 1 ;
- la figure 4 est une vue de dessus d'une variante du dispositif de la figure 1 ;
- la figure 5 est une vue en perspective d'un dispositif de surveillance d'une prothèse médicale et du corps humain ne faisant pas partie de la présente invention ;
- la figure 6 est une vue schématique de côté en coupe d'un dispositif de surveillance d'une prothèse médicale et du corps humain, selon un troisième mode de réalisation de l'invention ;
- la figure 7 est une vue en perspective du dispositif de la figure 6 ;
- la figure 8 est une vue schématique de côté en coupe d'une variante du dispositif de la figure 6 ;
- la figure 9 est une vue schématique illustrant un exemple de fonctionnement d'un dispositif de surveillance d'une prothèse médicale et du corps humain selon l'invention ; et
- les figures 10 et 11 sont des vues schématiques illustrant des exemples d'implantation du dispositif des figures 6 à 8.

### DESCRIPTION DES MODES DE REALISATION PREFERES

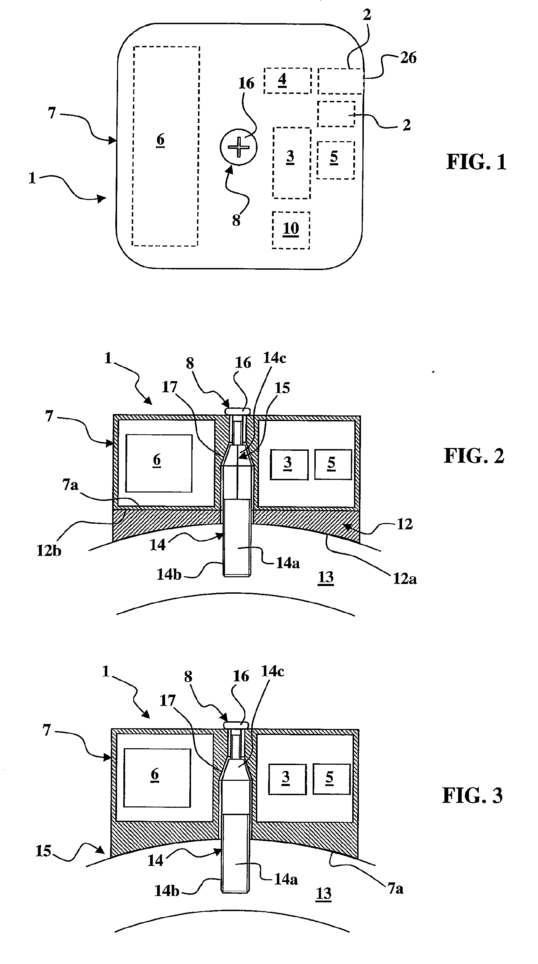

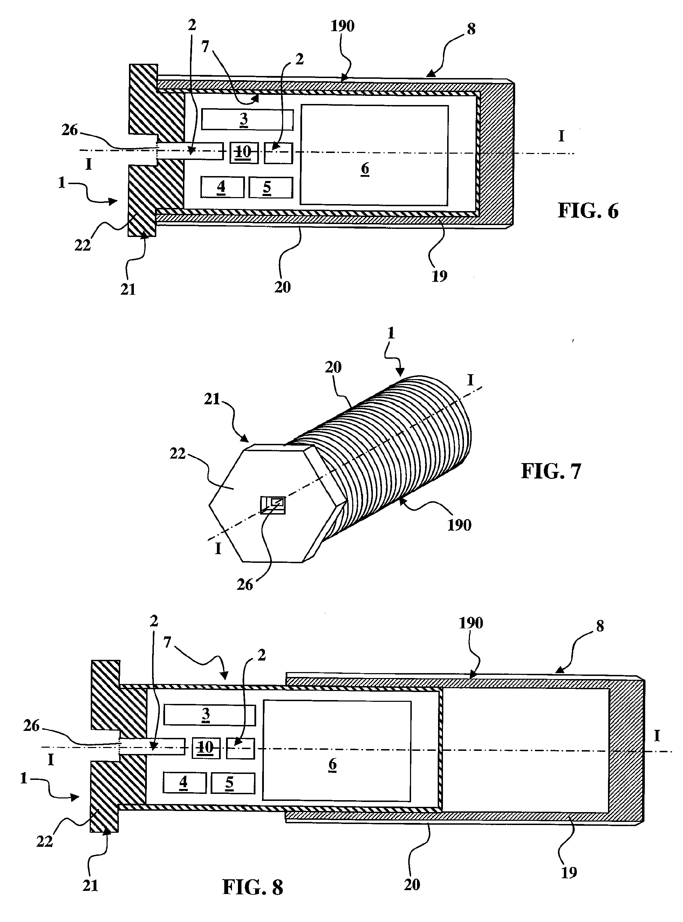

Les figures 1 et 6 illustrent respectivement deux modes de réalisation du dispositif de surveillance d'une prothèse médicale et du corps humain. Sur chacune de ces figures, on distingue un dispositif 1 de surveillance d'une prothèse médicale et du corps humain, comprenant :
- des moyens de capteur 2, aptes à générer des signaux en fonction de sollicitations environnantes,
- des moyens de traitement 3, aptes à traiter les signaux émis par les moyens de capteur 2 pour les convertir en données,
- des moyens de mémorisation 4, aptes à mémoriser les données,
- des moyens de transmission 5 sans fil des données mémorisées,
- des moyens d'alimentation 6 en énergie électrique,
- un boîtier 7 étanche en matériau biocompatible, dans lequel sont inclus les moyens de capteur 2, de traitement 3, de mémorisation 4, de transmission 5 et d'alimentation 6,
- le boîtier 7 comportant des moyens de fixation 8 à un os.

Des moyens de commande (non représentés) pilotent un ou plusieurs des moyens de capteur 2, des moyens de traitement 3, des moyens de mémorisation 4, des moyens de transmission 5 et des moyens d'alimentation 6.

Des exemples de matériaux biocompatibles pour constituer le boîtier 7 étanche sont : du titane, de l'alliage de titane, de l'acier inoxydable, du polymère (notamment le PEEK).

Dans une variante non représentée sur les figures, le dispositif 1 comporte au moins un premier boîtier, contenant les moyens d'alimentation 6 en énergie électrique, et qui est relié à un deuxième boîtier par des moyens de conduction électrique.

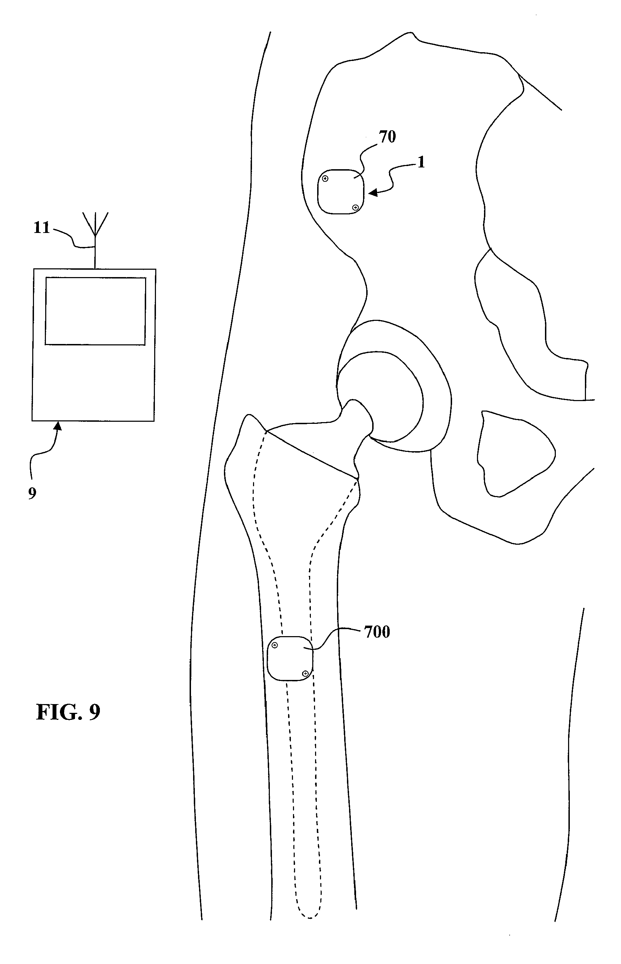

Dans la variante illustrée sur la figure 9, le dispositif 1 comporte :
- un premier boîtier 70 et un deuxième boîtier 700,
- au moins l'un des deux boîtiers 70 ou 700 comporte des moyens de capteur 2 (non visibles) adaptés pour détecter la position relative de l'autre boîtier.

Pour de tels moyens de capteur 2, on pourra notamment avoir recours aux enseignements du document WO 2010/003824.

Afin d'exploiter les données mémorisées, celles-ci sont transmises à un terminal distant 9 muni de moyens de réception 11. Le terminal distant 9 et les moyens de réception 11 sont adaptés pour recevoir directement les données mémorisées dans le premier boîtier 70 par les moyens de transmission du boîtier 70 et pour recevoir directement les données mémorisées dans le deuxième boîtier 700 par les moyens de transmission du deuxième boîtier 700.

Il est cependant possible qu'une transmission directe entre le premier boîtier 70 ou le deuxième boîtier 700 et le terminal distant 9 soit sujette à un défaut de propagation d'ondes. Par exemple, des tissus graisseux peuvent absorber une partie importante des ondes et ainsi empêcher la transmission des données mémorisées directement depuis l'un des boîtiers 70 ou 700 au terminal distant 9. Dans ce cas, il est avantageux de prévoir que :
- le dispositif 1 comporte des moyens de communication entre les premier et deuxième boîtiers 70 et 700, permettant à l'un et/ou l'autre des boîtiers 70 ou 700 de recevoir des données de l'autre boîtier 70 ou 700,
- au moins l'un des boîtiers 70 ou 700 comporte des moyens de transmission sans fil des données stockées pour émettre les données reçues de l'autre boîtier 70 ou 700.

Le premier boîtier 70 et/ou le deuxième boîtier 700 peuvent ainsi jouer un rôle de relai dans la transmission des données mémorisées contenues dans l'autre des boîtiers 70 ou 700.

Sur la figure 2 est illustrée une variante du dispositif de la figure 1 comportant une cale 12 qui comprend :
- une première face 12a destinée à venir en appui contre l'os 13 au moyen d'une conformation adaptée à la forme extérieure de l'os 13,
- une deuxième face 12b, sensiblement opposée à la première face 12a, destinée à venir en appui contre une face 7a du boîtier 7 au moyen d'une conformation adaptée à la face 7a du boîtier 7.

Le boîtier 7 vient ainsi indirectement en contact avec l'os 13 de façon intime.

Dans une autre variante illustrée sur la figure 3, le boîtier 7 peut lui-même comporter une face 7a destinée à venir en appui contre l'os 13 au moyen d'une conformation adaptée à la forme extérieure de l'os 13.

La variante de la figure 2 présente l'avantage, par rapport à la variante de la figure 3, de n'avoir à changer que la cale 12 en fonction de la forme de l'os, en vue de fixer un unique boîtier 7 de forme standardisée.

Sur la figure 2, on voit que les moyens de fixation 8 du boîtier 7 comprennent :
- une unique vis de solidarisation 14 du boîtier 7 à l'os 13,
- des moyens de blocage en rotation 15 du boîtier 7 par rapport à la vis de solidarisation 14.

Pour ce faire, on prévoit que :
- la vis de solidarisation 14 comprend une partie inférieure 14a à filet 14b à moyens d'ostéo-intégration,
- la vis de solidarisation 14 comprend une partie supérieure 14c comportant des moyens de verrouillage 16 du boîtier 7 sur la partie supérieure 14c.

En pratique, on voit sur la figure 2 que la partie supérieure 14c de la vis de solidarisation 14 présente une section non circulaire coopérant par complémentarité de formes avec un logement 17 prévu dans le boîtier 7 pour recevoir la partie supérieure 14c.

En alternative, comme représenté sur la figure 3, les moyens de fixation 8 du boîtier 7 peuvent comprendre :
- une unique vis de solidarisation 14 du boîtier 7 à l'os 13,
- des moyens de blocage en rotation 15 du boîtier 7 par rapport à l'os 13.

Sur la figure 3, les moyens de blocage en rotation 15 sont constitués par la face 7a du boîtier 7 venant en appui contre l'os 13. Il n'est donc pas besoin, dans ce cas, que la partie supérieure 14c de la vis de solidarisation 14 coopère par complémentarité de formes avec le logement 17 du boîtier 7 destiné à recevoir la partie supérieure 14c.

Il est à noter que, dans le cas des figures 2 et 3, la vis de solidarisation 14 est fixée de manière définitive dans l'os 13 mais le boîtier 7 peut librement être retiré pour être soumis à un remplacement ou à une maintenance. Le démontage du boîtier 7 s'effectue en dévissant les moyens de verrouillage 16 qui sont réversibles et se présentent sous forme d'une vis engagée par vissage dans la partie supérieure 14c de la vis de solidarisation 14. Le démontage du boîtier 7 pour son remplacement ou sa maintenance n'affecte donc pas la masse osseuse du patient qui se développera et se consolidera paisiblement autour de la vis de solidarisation 14.

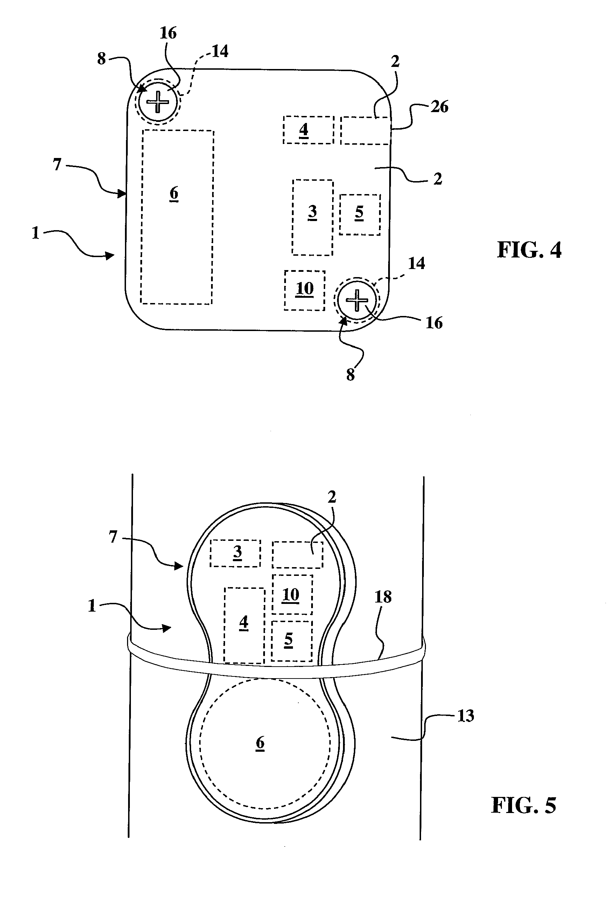

Dans une variante illustrée sur la figure 4, le boîtier 7 est fixé à un os par l'intermédiaire de moyens de fixation 8 comprenant deux vis de solidarisation 14 dont les parties supérieures 14c sont dépourvues de moyens de blocage en rotation susceptibles de bloquer le boîtier en rotation par rapport à l'os. Ces deux vis de solidarisation 14 permettent simultanément de solidariser le boîtier 7 à l'os et d'assurer une orientation constante du boîtier 7 par rapport à l'os sur lequel il est fixé.

En fonction de l'épaisseur de la masse osseuse et de l'état de cette masse osseuse, il peut être impossible d'avoir recours à des moyens de fixation 8 par vis. Dans ce cas, on a recours à un dispositif 1 illustré sur la figure 5 (qui ne fait pas partie de la présente invention), qui comprend un boîtier 7 en forme de cacahuète ou de « 8 » et fixé à l'os 13 par l'intermédiaire d'un lien souple 18 enserrant l'os 13.

Le lien souple 18 et la forme du boîtier 7 coopèrent pour assurer une orientation constante du boîtier 7 par rapport à l'os 13. Il est à noter que d'autres formes extérieures du boîtier 7 pourraient être utilisées en coopération avec le lien souple 18 pour garantir une orientation constante du boîtier 7 par rapport à l'os 13.

Un troisième mode de réalisation de l'invention est illustré sur les figures 6 et 7.

Sur ces figures, on voit que :
- le boîtier 7 est un boîtier tubulaire creux s'étendant selon une direction longitudinale I-I, apte à être verrouillé dans un manchon sensiblement tubulaire 190 à paroi extérieure 19 munie d'un filetage extérieur 20,
- le boîtier tubulaire comporte une extrémité 21 munie de moyens d'entraînement en rotation 22 autour de la direction longitudinale I-I.

En l'espèce, les moyens d'entraînement en rotation 22 comprennent une tête hexagonale permettant de visser le boîtier 7 dans la masse osseuse du patient.

On voit plus particulièrement sur la figure 8 que les moyens de fixation 8 comportent le manchon sensiblement tubulaire 190 à paroi extérieure 19 munie d'un filetage extérieur 20. Le boîtier 7 est rapporté dans le manchon tubulaire 190 par engagement en force et/ou par complémentarité de formes (vissage ou clipsage par exemple), ou par tous autres moyens de verrouillage réversibles convenables, de telle sorte que l'entraînement en rotation selon la direction longitudinale I-I du boîtier 7 par l'intermédiaire des moyens d'entraînement en rotation 22 entraîne simultanément en rotation la paroi extérieure 19 de façon à pénétrer par vissage dans une masse osseuse.

Il est ainsi possible de retirer le boîtier 7 et son contenu afin d'en effectuer une maintenance ou un remplacement sans avoir pour autant à extraire la paroi extérieure 19 filetée qui peut rester à demeure dans la masse osseuse du patient qui ne sera donc pas dégradée par la maintenance ou le remplacement.

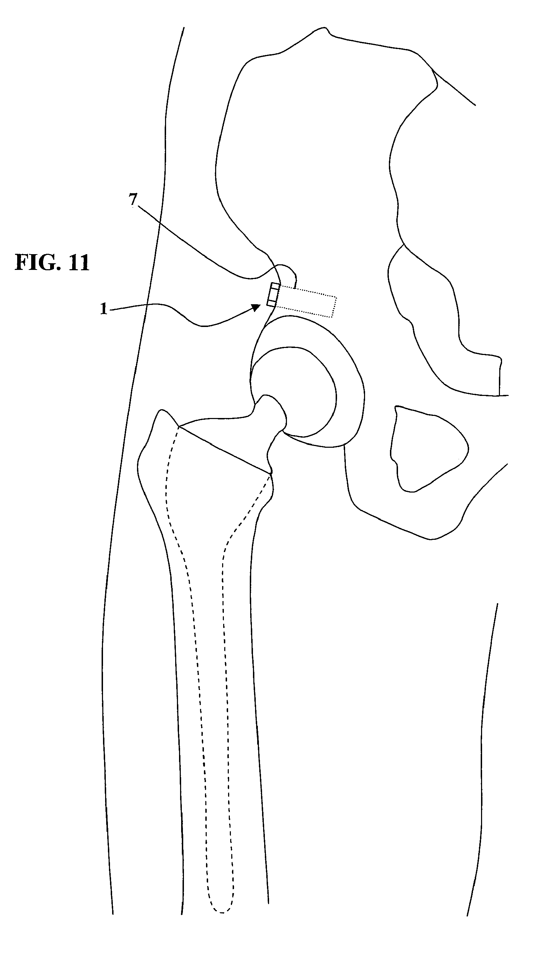

Dans la variante illustrée sur les figures 6 à 8, le dispositif 1 selon l'invention se présente sous la forme extérieure d'une vis. Cette vis pourra être implantée par exemple dans l'os iliaque du bassin du patient comme représenté sur la figure 11.

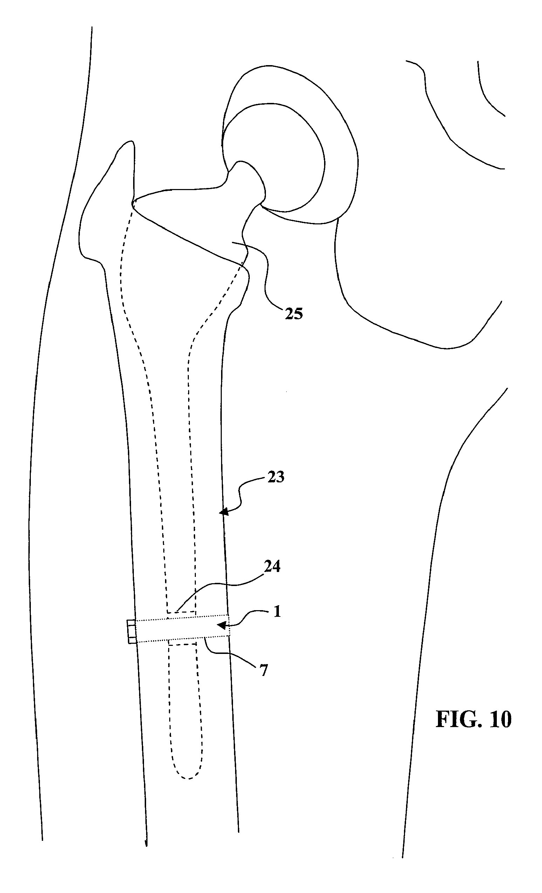

Le dispositif 1 des figures 6 à 8 pourra cependant en outre remplir des fonctions de vis de verrouillage comme illustré sur la figure 10 où le boîtier fileté 7 est vissé dans le fémur 23 du patient en passant à travers une lumière 24 prévue dans une tige fémorale 25 de prothèse de hanche. Le boîtier 7 à filetage externe remplit ainsi une fonction de verrouillage axial de la tige fémorale 25. Une telle implantation dans le fémur 23 est également possible sans toutefois assurer de verrouillage axial d'une tige fémorale.

Dans tous les modes de réalisation décrits précédemment, les moyens de capteur 2 peuvent être sensibles à des sollicitations électriques, chimiques, mécaniques ou optiques.

Dans le cas de moyens de capteur 2 ayant besoin d'effectuer des mesures dans le milieu environnant, tel qu'un capteur de conductivité, de température, ou de pH, on pourra prévoir de loger ledit moyen de capteur 2 à proximité immédiate d'une paroi du boîtier 7, ledit moyen de capteur 2 étant en contact avec le milieu environnant par l'intermédiaire d'une portion de paroi perméable 26 (figures 1 et 4).

Sur les figures 1, 4, 5, 6 et 8, le dispositif 1 comporte des moyens d'inhibition 10 pour inhiber sélectivement les moyens de capteur 2 et/ou les moyens de traitement 3.

Dans un premier mode de fonctionnement, les moyens d'inhibition 10 sont conformés pour inhiber le fonctionnement des moyens de capteur 2 et/ou des moyens de traitement 3 dans des plages horaires prédéterminées. Par exemple, les moyens d'inhibition 10 peuvent inhiber le fonctionnement des moyens de capteur 2 et/ou des moyens de traitement 3 pendant les heures de la nuit pour des personnes généralement inactives la nuit.

En alternative ou en complément, les moyens d'inhibition 10 sont conformés pour n'autoriser le fonctionnement des moyens de traitement 3 qu'après que les moyens de capteur 2 aient détecté des mouvements pendant une durée supérieure à une durée prédéterminée.

Un tel mode de fonctionnement permet de limiter efficacement la quantité de signaux émis par les moyens de capteur 2 à traiter par les moyens de traitement 3. Un tel mode de fonctionnement permet en outre de limiter les données mémorisées en excluant une quantité importante de mouvements de faible durée qui ne sont pas susceptibles de générer une usure sensible de la prothèse. Et cette limitation de données facilite et fiabilise une exploitation statistique des données pour en tirer des enseignements cliniques.

En alternative ou en complément, les moyens d'inhibition 10 sont conformés pour n'autoriser le fonctionnement des moyens de traitement 3 qu'après que les moyens de capteur 2 aient détecté des mouvements d'amplitude supérieure à une amplitude prédéterminée.

L'utilisation des moyens d'inhibition 10 décrits précédemment, permet de limiter le nombre de données à traiter, d'économiser de l'énergie électrique afin d'augmenter la durée de vie du dispositif 1 tout en permettant de fiabiliser les données statistiques qui pourront être établies sur la base des données mémorisées puis transmises.

Afin d'affiner le suivi clinique des patients et de mieux comprendre les éventuels dysfonctionnements des prothèses, on prévoit que :
- les moyens de mémorisation 4 des données contiennent des données de référence prédéterminées,
- le dispositif 1 comporte des moyens de comparaison, adaptés pour comparer aux données de référence prédéterminées les données issues du traitement des signaux émis par les moyens de capteur 2,
- en cas de non-conformité des données issues du traitement des signaux émis par les moyens de capteur 2 avec les données de référence prédéterminées, un signal d'anomalie est généré et stocké dans les moyens de mémorisation 4.

Les données de référence prédéterminées pourront par exemple consister en des amplitudes de mouvements, des angles de mouvements ou encore des vitesses angulaires d'exécution de mouvements. On pourra ainsi surveiller, au cours du temps, que la prothèse initialement prévue et implantée convient toujours au mode de vie et au comportement du patient. Et on pourra également vérifier de façon continue la conformité du fonctionnement de la prothèse ou de son milieu environnant pour détecter tout écart par rapport à une situation estimée normale correspondant aux données de référence prédéterminées.

Il est expressément précisé que les différents moyens de capteur 2, les moyens d'alimentation en énergie électrique, les différents moyens d'économie d'énergie mais également les différentes méthodes de traitement des données en comparaison avec des données de référence forment des inventions distinctes des moyens de fixation et de verrouillage et pourront faire l'objet d'une protection indépendante.

Pour réaliser les moyens de capteur 2 et les moyens de traitement 3, on pourra avoir recours aux enseignements des documents EP 2 106 747, WO 2010/097422, WO 2010/112469 et WO 2010/112470.

La présente invention n'est pas limitée aux modes de réalisation qui ont été explicitement décrits, mais elle en inclut les diverses variantes et généralisations contenues dans le domaine des revendications ci-après.

## Revendications

1. Dispositif (1) de surveillance d'une prothèse médicale et du corps humain, comprenant :
- des moyens de capteur (2), aptes à générer des signaux en fonction de sollicitations environnantes,
- des moyens de traitement (3), aptes à traiter les signaux émis par les moyens de capteur (2) pour les convertir en données,
- des moyens de mémorisation (4), aptes à mémoriser les données,
- des moyens de transmission (5) sans fil des données mémorisées,
- des moyens d'alimentation (6) en énergie électrique,
dans lequel lesdits moyens de capteur (2), de traitement (3), de mémorisation (4), de transmission (5) et d'alimentation (6) sont inclus dans au moins un boîtier (7) en matériau biocompatible,
**caractérisé en ce que** ledit au moins un boîtier (7) est solidarisé de façon réversible à des moyens de fixation (8, 14, 19) par des moyens de verrouillage (16), lesdits moyens de fixation (8) étant destinés et adaptés pour être fixés à un os.

2. Dispositif (1) selon la revendication 1, **caractérisé en ce que** ledit au moins un boîtier (7, 70, 700) comporte une face (7a) destinée à venir en appui contre l'os (13) au moyen d'une conformation adaptée à la forme extérieure de l'os (13).

3. Dispositif (1) selon l'une des revendications 1 ou 2, **caractérisé en ce qu'**il comporte une cale (12) comprenant :
- une première face (12a) destinée à venir en appui contre l'os (13) au moyen d'une conformation adaptée à la forme extérieure de l'os (13),
- une deuxième face (12b), sensiblement opposée à la première face (12a), destinée à venir en appui contre une face (7a) dudit au moins un boîtier (7, 70, 700) au moyen d'une conformation adaptée à la face (7a) dudit au moins un boîtier (7, 70, 700).

4. Dispositif (1) selon l'une quelconque des revendications 1 à 3, **caractérisé en ce que** les moyens de fixation (8) dudit au moins un boîtier (7, 70, 700) comprennent :
- une unique vis de solidarisation (14) dudit au moins un boîtier (7, 70, 700) à l'os (13),
- des moyens de blocage en rotation (15) dudit au moins un boîtier (7, 70, 700) par rapport à la vis de solidarisation (14) ou par rapport à l'os (13).

5. Dispositif (1) selon l'une quelconque des revendications 1 à 4, **caractérisé en ce que** :
- les moyens de fixation (8) comportent au moins une vis de solidarisation (14) ou, le cas échéant, une unique vis de solidarisation (14),
- la vis de solidarisation (14) comprend une partie inférieure (14a) à filet (14b) à moyens d'ostéo-intégration,
- la vis de solidarisation (14) comprend une partie supérieure (14c) adaptée pour coopérer avec les moyens de verrouillage (16) dudit au moins un boîtier (7, 70, 700) sur la partie supérieure (14c) afin de retenir ledit boîtier (7, 70, 700).

6. Dispositif (1) selon la revendication 1, **caractérisé en ce que** :
- ledit au moins un boîtier (7, 70, 700) est un boîtier tubulaire creux, s'étendant selon une direction longitudinale (I-I), apte à être verrouillé dans un manchon sensiblement tubulaire (190) à paroi extérieure (19) munie d'un filetage extérieur (20),
- le boîtier tubulaire et/ou manchon sensiblement tubulaire (190) comporte une extrémité (21) munie de moyens d'entraînement en rotation (22) autour de la direction longitudinale (1-1).

7. Dispositif (1) selon l'une quelconque des revendications 1 à 6, **caractérisé en ce qu'**il comporte au moins un premier boîtier (70), contenant les moyens d'alimentation (6) en énergie électrique, et qui est relié à un deuxième boîtier (700) par des moyens de conduction électrique.

8. Dispositif (1) selon l'une quelconque des revendications 1 à 7, **caractérisé en ce que** :
- il comporte au moins un premier boîtier (70) et un deuxième boîtier (700),
- au moins l'un des deux boîtiers (70, 700) comporte des moyens de capteur (2) adaptés pour détecter la position relative de l'autre boîtier (70, 700).

9. Dispositif (1) selon l'une quelconque des revendications 1 à 8, **caractérisé en ce que** :
- il comporte au moins un premier (70) et un deuxième boîtiers (700),
- il comporte des moyens de communication entre les premier et deuxième boîtiers (70, 700), permettant à l'un et/ou l'autre des boîtiers (70, 700) de recevoir des données de l'autre boîtier (70, 700),
- au moins l'un des boîtiers (70, 700) comporte des moyens de transmission (5) sans fil des données stockées pour émettre les données reçues de l'autre boîtier (70, 700).

10. Dispositif (1) selon l'une quelconque des revendications 1 à 9, **caractérisé en ce que** les moyens de capteur (2) sont sensibles à des sollicitations électriques, chimiques, mécaniques ou optiques.

11. Dispositif (1) selon l'une quelconque des revendications 1 à 10, **caractérisé en ce que** les moyens de capteur (2) comportent un gyromètre.

12. Dispositif (1) selon l'une quelconque des revendications 1 à 11, **caractérisé en ce que** les moyens de capteur (2) comportent un inclinomètre, de préférence de type magnétomètre.

13. Dispositif (1) selon l'une quelconque des revendications 1 à 12, **caractérisé en ce que** les moyens de capteur (2) comportent un accéléromètre.

14. Dispositif (1) selon l'une quelconque des revendications 1 à 13, **caractérisé en ce que** les moyens de capteur (2) comportent un capteur de pression.

15. Dispositif (1) selon l'une quelconque des revendications 1 à 14, **caractérisé en ce que** les moyens de capteur (2) comportent un capteur de mesure de la conductivité du milieu environnant.

16. Dispositif (1) selon l'une quelconque des revendications 1 à 15, **caractérisé en ce que** les moyens de capteur (2) comportent un capteur de température.

17. Dispositif (1) selon l'une quelconque des revendications 1 à 16, **caractérisé en ce que** les moyens de capteur (2) comportent un capteur de mesure du pH.

18. Dispositif (1) selon l'une quelconque des revendications 1 à 17, **caractérisé en ce que** les moyens de capteur (2) comportent un capteur optique adapté pour détecter la couleur du milieu environnant.

19. Dispositif (1) selon l'une quelconque des revendications 1 à 18, **caractérisé en ce que** les moyens d'alimentation (6) en énergie électrique comprennent :
- des moyens de conversion des accélérations subies par ledit au moins un boîtier (7, 70, 700) pour les convertir en énergie électrique,
- des moyens de stockage de l'énergie électrique convertie.

20. Dispositif (1) selon l'une quelconque des revendications 1 à 19, **caractérisé en ce que** :
- le dispositif (1) comporte des moyens d'inhibition (10) de certains moyens de capteur (2) et/ou moyens de traitement (3),
- les moyens d'inhibition (10) sont conformés pour inhiber le fonctionnement de ces moyens de capteur (2) et/ou moyens de traitement (3) pendant des plages horaires prédéterminées.

21. Dispositif (1) selon l'une quelconque des revendications 1 à 20, **caractérisé en ce que** :
- il comporte des moyens d'inhibition (10) de certains moyens de capteur (2) et/ou moyens de traitement (3),
- les moyens d'inhibition (10) sont conformés pour n'autoriser le fonctionnement des moyens de traitement (3) qu'après que des moyens de capteur (2) non inhibés aient détecté des mouvements pendant une durée supérieure à une durée prédéterminée.

22. Dispositif (1) selon l'une quelconque des revendications 1 à 21, **caractérisé en ce que** :
- le dispositif (1) comporte des moyens d'inhibition (10) de certains moyens de capteur (2) et/ou moyens de traitement (3),
- les moyens d'inhibition (10) sont conformés pour n'autoriser le fonctionnement des moyens de traitement (3) qu'après que des moyens de capteur (2) non inhibés aient détecté des mouvements d'amplitude supérieure à une amplitude prédéterminée.

23. Dispositif (1) selon l'une quelconque des revendications 1 à 22, **caractérisé en ce que** :
- les moyens de mémorisation (4) des données contiennent des données de référence prédéterminées,
- le dispositif (1) comporte des moyens de comparaison, adaptés pour comparer aux données de référence prédéterminées les données issues du traitement des signaux émis par les moyens de capteur (2),
- en cas de non-conformité des données issues du traitement des signaux émis par les moyens de capteur (2) avec les données de référence prédéterminées, un signal d'anomalie est généré et stocké dans les moyens de mémorisation (4).

## Patentansprüche

1. Vorrichtung (1) zur Überwachung eines medizinische Prothese und des menschlichen Körpers, umfassend:
- Sensormittel (2), die ausgebildet sind, Signale in Abhängigkeit von Umgebungsbeanspruchungen zu erzeugen,
- Verarbeitungsmittel (3), die ausgebildet sind, die von den Sensormitteln (2) ausgegebenen Signale zu behandeln, um sie in Daten umzuwandeln,
- Speichermittel (4), die ausgebildet sind, die Daten zu speichern,
- drahtlose Übertragungsmittel (5) der gespeicherten Daten,
- Versorgungsmittel (6) für elektrische Energie, wobei die genannten Sensormittel (2), Verarbeitungsmittel (3), Speichermittel (4), Übertragungsmittel (5) und Versorgungsmittel (6) in mindestens einem Gehäuse (7) aus biokompatiblen Materialien enthalten sind,
**dadurch gekennzeichnet, dass** das genannte mindestens ein Gehäuse (7) in reversibler Weise mit Befestigungsmitteln (8, 14, 19) über Verriegelungsmittel (16) verbunden ist, wobei die genannten Befestigungsmittel (8) dazu bestimmt und ausgebildet sind, an einem Knochen befestigt zu werden.

2. Vorrichtung (1) nach Anspruch 1, **dadurch gekennzeichnet, dass** das mindestens eine Gehäuse (7, 70, 700) eine Fläche (7a) aufweist, die dazu bestimmt ist, gegen den Knochen (13) mittels einer Ausformung in Anschlag zu kommen, die an die äußere Form des Knochens (13) angepasst ist.

3. Vorrichtung (1) nach einem der Ansprüche 1 oder 2, **dadurch gekennzeichnet, dass** sie eine Zwischenscheibe (12) enthält, die aufweist:
- eine erste Fläche (12a), die dazu bestimmt ist, gegen den Knochen (13) mit einer Ausformung in Anschlag zu kommen, die an die äußere Form des Knochens (13) angepasst ist,
- eine zweite Fläche (12b), die im wesentlichen der ersten Fläche (12a) gegenüberliegt und dazu bestimmt ist, gegen eine Fläche (7a) des genannten mindestens einen Gehäuses (7, 70, 700) mit einer Ausformung in Anschlag zu kommen, die an die Fläche (7a) des genannten mindestens einen Gehäuses (7, 70, 700) angepasst ist.

4. Vorrichtung (1) nach irgendeinem der Ansprüche 1 bis 3, **dadurch gekennzeichnet, dass** die Befestigungsmittel (8) des genannten mindestens einen Gehäuses (7, 70, 700) aufweisen:
- eine einzige Befestigungsschraube (14) des genannten mindestens einen Gehäuses (7, 70, 700) am Knochen (13),
- Mittel zum Blockieren in Drehrichtung (15) des genannten mindestens einen Behälters (7, 70, 700) relativ zur Befestigungsschraube (14) oder relativ zu dem Knochen (13).

5. Vorrichtung (1) nach irgendeinem der Ansprüche 1 bis 4, **dadurch gekennzeichnet, dass**:
- die Befestigungsmittel (8) mindestens eine Verbindungsschraube (14) oder gegebenenfalls eine einzige Verbindungsschraube (14) aufweisen,
- die Befestigungsschraube (14) einen unteren Teil (14a) mit Gewinde (14b) mit osteo-Integrationsmitteln aufweist,
- die Verbindungsschraube (14) ein oberes Teil (14c) aufweist, das ausgebildet ist, um mit den Verriegelungsmitteln (16) des genannten mindestens einen Gehäuses (7, 70, 700) an dem oberen Teil (14c) zu kooperieren, um das genannte Gehäuse (7, 70, 700) zu halten.

6. Vorrichtung (1) nach Anspruch 1, **dadurch gekennzeichnet, dass**:
- das genannte mindestens eine Gehäuse (7, 70, 700) ein hohles rohrförmiges Gehäuse ist, das sich in einer Längsrichtung (I-I) erstreckt und ausgebildet ist, in einer im wesentlichen rohrförmigen Hülse (190) an einer Aussenwand (19) verriegelt zu werden, die mit einem Aussengewinde (20) versehen ist,
- das rohrförmige Gehäuse und/oder die im wesentlichen rohrförmige Hülse (190) ein Ende (21) aufweisen, die mit Mitteln (22) zum Drehantrieb um die Längsrichtung (I-I) versehen sind.

7. Vorrichtung (1) nach irgendeinem der Ansprüche 1 bis 6, **dadurch gekennzeichnet, dass** sie mindestens ein erstes Gehäuse (70) aufweist, das die Versorgungsmittel (6) mit elektrischer Energie enthält und das mit einem zweiten Gehäuse (700) über Mittel zur elektrischen Verbindung verbunden ist.

8. Vorrichtung (1) nach irgendeinem der Ansprüche 1 bis 7, **dadurch gekennzeichnet, dass**:
- sie mindestens ein erstes Gehäuse (70) und ein zweites Gehäuse (700) aufweist,
- mindestens eines der beiden Gehäuse (70, 700) Sensormittel (2) enthält, die ausgebildet sind, um die relative Position des anderen Gehäuses (70, 700) zu erfassen.

9. Vorrichtung (1) nach irgendeinem der Ansprüche 1 bis 8, **dadurch gekennzeichnet, dass**:
- sie mindestens ein erstes (70) und ein zweites Gehäuse (700) aufweist,
- sie Kommunikationsmittel zwischen dem ersten und dem zweiten Gehäuse (70, 700) aufweist, die dem einen oder dem anderen der Gehäuse (70, 700) erlauben, die Daten des anderen Gehäuses (70, 700) zu empfangen,
- mindestens eines der Gehäuse (70, 700) drahtlose Übertragungsmittel (5) für gespeicherte Daten aufweist, um die empfangenen Daten des anderen Gehäuses (70, 700) zu übertragen.

10. Vorrichtung (1) nach irgendeinem der Ansprüche 1 bis 9, **dadurch gekennzeichnet, dass** die Sensormittel (2) für elektrische, chemische, mechanische oder optische Anregungen sensibel sind.

11. Vorrichtung (1) nach irgendeinem der Ansprüche 1 bis 10, **dadurch gekennzeichnet, dass** die Sensormittel (2) ein Gyrometer aufweisen.

12. Vorrichtung (1) nach irgendeinem der Ansprüche 1 bis 11, **dadurch gekennzeichnet, dass** die Sensormittel (2) ein Inclinometer aufweisen, vorzugsweise vom Typ eines Magnetometers.

13. Vorrichtung (1) nach irgendeinem der Ansprüche 1 bis 12, **dadurch gekennzeichnet, dass** die Sensormittel (2) einen Beschleunigungsmesser aufweisen.

14. Vorrichtung (1) nach irgendeinem der Ansprüche 1 bis 13, **dadurch gekennzeichnet, dass** die Sensormittel (2) Druckmesser aufweisen.

15. Vorrichtung (1) nach irgendeinem der Ansprüche 1 bis 14, **dadurch gekennzeichnet, dass** die Sensormittel (2) einen Sensor zum Messen der Konduktivität des Umgebungsmillieus aufweisen.

16. Vorrichtung (1) nach irgendeinem der Ansprüche 1 bis 15, **dadurch gekennzeichnet, dass** die Sensormittel (2) einen Temperatursensor aufweisen.

17. Vorrichtung (1) nach irgendeinem der Ansprüche 1 bis 16, **dadurch gekennzeichnet, dass** die Sensormittel (2) einen Sensor zum Messen eines pH-Wertes aufweisen.

18. Vorrichtung (1) nach irgendeinem der Ansprüche 1 bis 17, **dadurch gekennzeichnet, dass** die Sensormittel (2) einen optischen Sensor aufweisen, der ausgebildet ist, die Farbe des Umgebungsmillieus zu erfassen.

19. Vorrichtung (1) nach irgendeinem der Ansprüche 1 bis 18, **dadurch gekennzeichnet, dass** die Mittel zur Versorgung (6) mit elektrischer Energie aufweisen:
- Mittel zur Umwandlung von Beschleunigungen, denen der genannte mindestens eine Behälter (7, 70, 700) unterworfen ist, um sie in elektrische Energie umzuwandeln,
- Mittel zum Speichern der umgewandelten elektrischen Energie.

20. Vorrichtung (1) nach irgendeinem der Ansprüche 1 bis 19, **dadurch gekennzeichnet, dass**:
- die Vorrichtung (1) Sperrmittel (10) aufweist, um einige der Sensormittel (2) und/oder der Verarbeitungsmittel (3) zu deaktivieren,
- die Sperrmittel (10) ausgebildet sind, die Funktion der Sensormittel (2) und/oder der Verarbeitungsmittel (3) während vorbestimmter Zeiten zu sperren.

21. Vorrichtung (1) nach irgendeinem der Ansprüche 1 bis 20, **dadurch gekennzeichnet, dass**:
- sie Sperrmittel (10) für einige der Sensormittel (2) und/oder der Verarbeitungsmittel (3) aufweist,
- die Sperrmittel (10) ausgebildet sind, um die Funktion der Verarbeitungsmittel (3) nicht zu autorisieren, nachdem die nicht gesperrten Sensormittel (2) Bewegungen während einer Zeitdauer erfasst haben, die größer ist als eine vorbestimmte Zeitdauer.

22. Vorrichtung (1) nach irgendeinem der Ansprüche 1 bis 21, **dadurch gekennzeichnet, dass**:
- die Vorrichtung (1) Sperrmittel (10) für einige der Sensormittel (2) und/oder Verarbeitungsmittel (3) aufweist,
- die Sperrmittel (10) ausgebildet sind, die Funktion der Verarbeitungsmittel (3) nicht zu autorisieren, nachdem die nicht gesperrten Sensormittel (2) Bewegungen mit einer Amplitude erfasst haben, die größer ist als eine vorbestimmte Amplitude.

23. Vorrichtung (1) nach irgendeinem der Ansprüche 1 bis 22, **dadurch gekennzeichnet, dass**:
- die Speichermittel (4) für Daten vorbestimmte Referenzdaten enthalten,
- die Vorrichtung (1) Vergleichsmittel enthält, die ausgebildet sind, von den Verarbeitungsmitteln für die von den Sensormitteln (2) ausgesandten Daten mit den vorbestimmten Referenzdaten zu vergleichen,
- im Falle einer nicht-Konformität der von den Verarbeitungsmitteln der von den Sensormitteln (2) ausgesandten Daten mit den vorbestimmten Referenzdaten ein Anomaliesignal erzeugt und in den Speichermitteln (4) gespeichert wird.

## Claims

1. Device (1) for monitoring a medical prosthesis and the human body, comprising:
- sensor means (2), capable of generating signals according to surrounding stresses,
- processing means (3), capable of processing the signals transmitted by the sensor means (2) to convert them into data,
- storage means (4), capable of storing the data,
- means (5) for wirelessly transmitting the stored data,
- electrical energy power supply means (6),
in which said sensor (2), processing (3), storage (4), transmission (5) and power supply (6) means are included in at least one housing (7) made of biocompatible material,
**characterized in that** said at least one housing (7) is secured reversibly to fixing means (8, 14, 19) by locking means (16), said fixing means (8) being intended and designed to be fixed to a bone.

2. Device (1) as claimed in claim 1, **characterized in that** said at least one housing (7, 70, 700) comprises a face (7a) intended to bear against the bone (13) by means of a shaping adapted to the external shape of the bone (13).

3. Device (1) as claimed in one of claims 1 or 2, **characterized in that** it comprises a shim (12) comprising:
- a first face (12a) intended to bear against the bone (13) by means of a shaping adapted to the external shape of the bone (13),
- a second face (12b), substantially opposite the first face (12a), intended to bear against a face (7a) of said at least one housing (7, 70, 700) by means of a shaping adapted to the face (7a) of said at least one housing (7, 70, 700).

4. Device (1) as claimed in any one of claims 1 to 3, **characterized in that** the fixing means (8) of said at least one housing (7, 70, 700) comprise:
- a single screw (14) for securing said at least one housing (7, 70, 700) to the bone (13),
- means for blocking the rotation (15) of said at least one housing (7, 70, 700) relative to the securing screw (14) or relative to the bone (13).

5. Device (1) as claimed in any one of claims 1 to 4, **characterized in that**:
- the fixing means (8) comprise at least one securing screw (14) or, if appropriate, a single securing screw (14),
- the securing screw (14) comprises a bottom part (14a) with thread (14b) with osteo-integration means,
- the securing screw (14) comprises a top part (14c) designed to cooperate with the locking means (16) of said at least one housing (7, 70, 700) on the top part (14c) in order to retain said housing (7, 70, 700).

6. Device (1) as claimed in claim 1, **characterized in that**:
- said at least one housing (7, 70, 700) is a hollow tubular housing, extending in a longitudinal direction (I-I), capable of being locked in a substantially tubular sleeve (190) with outer wall (19) provided with an outer threading (20),
- the tubular housing and/or substantially tubular sleeve (190) comprises an end (21) provided with means (22) for driving in rotation about the longitudinal direction (I-I).

7. Device (1) as claimed in any one of claims 1 to 6, **characterized in that** it comprises at least one first housing (70), containing the electrical energy power supply means (6), and which is linked to a second housing (700) by electrical conduction means.

8. Device (1) as claimed in any one of claims 1 to 7, **characterized in that**:
- it comprises at least one first housing (70) and one second housing (700),
- at least one of the two housings (70, 700) comprises sensor means (2) designed to detect the relative position of the other housing (70, 700).

9. Device (1) as claimed in any one of claims 1 to 8, **characterized in that**:
- it comprises at least one first housing (70) and one second housing (700),
- it comprises means for communication between the first and second housings (70, 700), enabling one and/or the other of the housings (70, 700) to receive data from the other housing (70, 700),
- at least one of the housings (70, 700) comprises means (5) for wirelessly transmitting the stored data to transmit the data received from the other housing (70, 700).

10. Device (1) as claimed in any one of claims 1 to 9, **characterized in that** the sensor means (2) are sensitive to electrical, chemical, mechanical or optical stresses.

11. Device (1) as claimed in any one of claims 1 to 10, **characterized in that** the sensor means (2) comprise a gyrometer.

12. Device (1) as claimed in any one of claims 1 to 11, **characterized in that** the sensor means (2) comprise an inclinometer, preferably of magnetometer type.

13. Device (1) as claimed in any one of claims 1 to 12, **characterized in that** the sensor means (2) comprise an accelerometer.

14. Device (1) as claimed in any one of claims 1 to 13, **characterized in that** the sensor means (2) comprise a pressure sensor.

15. Device (1) as claimed in any one of claims 1 to 14, **characterized in that** the sensor means (2) comprise a sensor for measuring the conductivity of the surrounding medium.

16. Device (1) as claimed in any one of claims 1 to 15, **characterized in that** the sensor means (2) comprise a temperature sensor.

17. Device (1) as claimed in any one of claims 1 to 16, **characterized in that** the sensor means (2) comprise a sensor for measuring the pH.

18. Device (1) as claimed in any one of claims 1 to 17, **characterized in that** the sensor means (2) comprise an optical sensor designed to detect the color of the surrounding medium.

19. Device (1) as claimed in any one of claims 1 to 18, **characterized in that** the electrical energy power supply means (6) comprise:
- means for converting accelerations undergone by said at least one housing (7, 70, 700) to convert them into electrical energy,
- means for storing the converted electrical energy.

20. Device (1) as claimed in any one of claims 1 to 19, **characterized in that**:
- the device (1) comprises means (10) for disabling some sensor means (2) and/or processing means (3),
- the disabling means (10) are configured to disable the operation of these sensor means (2) and/or processing means (3) during predetermined time bands.

21. Device (1) as claimed in any one of claims 1 to 20, **characterized in that**:
- it comprises means (10) for disabling some sensor means (2) and/or processing means (3),
- the disabling means (10) are configured to allow the operation of the processing means (3) only after the non-disabled sensor means (2) have detected movements for a duration greater than a predetermined duration.

22. Device (1) as claimed in any one of claims 1 to 21, **characterized in that**:
- the device (1) comprises means (10) for disabling some sensor means (2) and/or processing means (3),
- the disabling means (10) are configured to allow the operation of the processing means (3) only after the non-disabled sensor means (2) have detected movements with an amplitude greater than a predetermined amplitude.

23. Device (1) as claimed in any one of claims 1 to 22, **characterized in that**:
- the data storage means (4) contain predetermined reference data,
- the device (1) comprises comparison means, designed to compare the data obtained from the processing of the signals transmitted by the sensor means (2) with the predetermined reference data,
- in the event of nonconformity of the data obtained from the processing of the signals transmitted by the sensor means (2) with the predetermined reference data, an anomaly signal is generated and stored in the storage means (4).
